# EUROPEAN PATENT SPECIFICATION

(11) **EP 4 124 571 B1**
(45) Date of publication and mention of the grant of the patent: **10.12.2025**
(21) Application number: 22187016.5
(22) Date of filing: 26.07.2022
(51) Int. Cl.: B64D 11/02

(54) **SANITIZATION IDENTIFICATION SYSTEMS AND METHODS**
SYSTEME UND VERFAHREN ZUR IDENTIFIZIERUNG VON HYGIENEMASSNAHMEN
SYSTÈMES ET PROCÉDÉS D'IDENTIFICATION D'ASSAINISSEMENT

(30) Priority: 28.07.2021 US 202117387340
(43) Date of publication of application: 01.02.2023
(73) Proprietor: B/E Aerospace, Inc., Winston-Salem, NC 27105 (US)
(72) Inventor: EDQUIST, John, Milkwaukee, WI (US); MARTZ, Thomas, Winston-Salem, NC (US); POTEET, Steven, Ashland, MA (US); McCONNELL, David, Annalong, Northern Ireland (GB); PIMENTEL, Katherina Urena, Manchester, CT (US)
(74) Representative: Dehns

(56) References cited:
- EP-A1- 3 421 054
- WO-A1-2020/226137
- US-A1- 2015 069 265
- US-A1- 2016 220 716
- US-A1- 2020 215 214
- US-A1- 2021 095 138
- US-A1- 2021 097 481

## Description

### FIELD

The present disclosure relates generally to sanitization systems and methods and, more particularly, to fluorescence based indicators for use in lighting sanitization systems.

### BACKGROUND

The recent novel-coronavirus (SARS-COV-2) outbreak has negatively impacted the safety and health of many people. Pathogens can be transmitted via direct airborne transmission between users or via indirect contact transmission from different users occupying the same space at different times. For example, lingering pathogens may remain on contact surfaces of an aircraft cabin to be spread to passengers and/or crew members on a subsequent flight. The safety of passengers and crew members may be improved by performing disinfecting treatments to surfaces, such as seats, ceiling/wall panels, handles. Additionally, providing indication that said treatment is in process could be beneficial. US 2021/097481 A1 describes a food locker sanitation system. EP 3 421 054 A1 relates to cleanliness indication systems and methods. US 2016/220716 A1 relates to a lavatory disinfection system.

### SUMMARY

A method of verifying sanitization is provided as defined by claim 1. The method in a lay-out not being part of the claimed subject-matter, may comprise: emitting, via a sanitization system, a UV-C light towards a desired surface to be sanitized, the UV-C light including a first wavelength between 200 nm and 280 nm; and determining the desired surface is being sanitized by verifying whether the desired surface absorbs the UV-C light and re-emits a visible light.

In various embodiments, the visible light includes a second wavelength between 380 nm and 750 nm. The visible light may be between 400 and 470 nm. The sanitization system may be a portable sanitization system. The desired surface may include a fluorescent brightener disposed thereon. The method may further comprise traversing the area.

A method of sanitization indication is disclosed herein. The method may comprise: initiation a sanitization process including emitting a UV-C light from a light source in a sanitization system; and providing an indication that the sanitization process is in progress, the indication being provided by a fluorescent brightener disposed on a surface.

In various embodiments, the fluorescent brightener is configured to: absorb the UV-C light; and re-emit a visible light to indicate an area is being sanitized. The area is a lavatory of an aircraft. The surface is on a wall in a lavatory being sanitized. The surface may be a desired surface to be sanitized in the area. The UV-C light includes a first wavelength between 200 nm and 280 nm. The visible light may include a second wavelength between 380 nm and 750 nm. The method may further comprise directing the UV-C light towards the surface throughout the sanitization process.

A sanitization indication system is provided as defined by claim 9. The sanitization indication system comprises: a surface configured to be disposed on a wall in the lavatory of the aircraft; a fluorescent brightener disposed on the surface, the fluorescent brightener configured to receive a UV-C light from a UV-C light source during a sanitization process.

In various embodiments, the sanitization indication system further comprises the UV-C light source. The surface may be disposed across from a second surface to be sanitized, the surface configured to act solely as an indication of the sanitization process. The UV-C light may be configured to emit the UV-C light that includes a first wavelength between 200 nm and 280 nm. The fluorescent brightener may be configured to absorb the UV-C light and re-emit a visible light having second wavelength between 380 nm and 750 nm

The forgoing features and elements may be combined in various combinations without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1 illustrates an aircraft cabin with a sanitization system, in accordance with various embodiments;
FIG. 2 illustrates a lavatory of an aircraft, in accordance with various embodiments;
FIG. 3 illustrates a sanitization indication system, in accordance with various embodiments;
FIG. 4 illustrates a process for using a sanitization indication system, in accordance with various embodiments;
FIG. 5 illustrates a method for using a sanitization indication system, in accordance with various embodiments; and
FIG. 6 illustrates a method for using a sanitization indication system, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the disclosure. Thus, the detailed description herein is presented for purposes of illustration only and not of limitation. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

UV-C light as disclosed herein refers to light having a wavelength between 200 nm and 280 nm, or more preferably approximately 222nm. UV-C lights can emit a blue hue that would be an indication of the unit being on if looking directly at a respective unit. However, looking at the light directly could damage an individual's eyesight. Additionally, an individual may not know where the light for a respective sanitization system is located. Thus, an individual entering a room being sanitized by UV-C may be unaware of the sanitization and could potentially be exposed to the UV-C for long durations.

Disclosed herein are sanitization indication systems and methods for use in sanitization systems configured for ultraviolet-C light (UV-C) light. In various embodiments, the sanitization indication system comprises a fluorescent dye. The fluorescent dye is at least partially invisible under standard lighting conditions but is configured to glow under UV-C light emittance. In this regard, the fluorescent dye may be disposed in locations configured for sanitization via a UV-C sanitization system to act as an indicator (i.e., an on/off activity sensor), in accordance with various embodiments. For example, the fluorescent dye indicator may be placed on a surface in the light's proximity informing the public of the sanitization system's activity. The indicator may be applied holistically to all surfaces to be cleaned, in a label/dot form, or even as an image or wording indicating sanitization of a respective room, in accordance with various embodiments.

In various embodiments, sanitization indication systems and methods disclosed herein may facilitate easy application of the indicator, low cost, a holistic view, long lasting properties, and/or not have electricity or power.

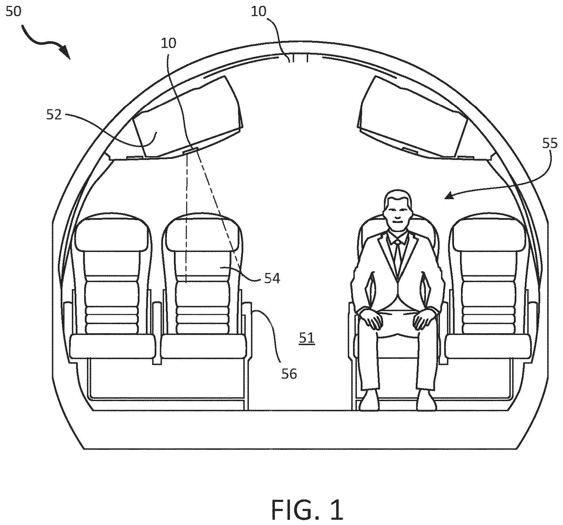

With reference to FIG. 1, a cabin 51 of an aircraft 50 is shown, according to various embodiments. The aircraft 50 may be any aircraft such as an airplane, a helicopter, or any other aircraft. The aircraft 50 may include various lighting systems 10 that emit visible light to the cabin 51. Pathogens, such as viruses and bacteria, may remain on surfaces of the cabin 51, and these remaining pathogens may result in indirect contact transmission to other people (e.g., subsequent passengers). For example, the cabin 51 may include overhead bins 52, passenger seats 54 for supporting passengers 55, handles 56, lavatory surfaces, and other structures/surfaces upon which active pathogens may temporarily reside. As will be discussed further below, in order to reduce the transmission/transfer of pathogens between passengers, one or more of the lighting systems 10 may blend disinfecting electromagnetic radiation output into the visible light in order to facilitate disinfection of the cabin 51 (e.g., during flights and/or between flights). The lighting systems 10 may be broken down into different addressable lighting regions that could be used on an aircraft. For example, the regions on an aircraft may include sidewall lighting, cross-bin lighting, over wing exit lighting, ceiling lighting, direct lighting, flex lights, reading lights, dome lights, lavatory lights, mirror lights, cockpit lights, cargo lights, etc. The regional breakdown of the lighting system allows lighting control over broad areas of the aircraft. In various embodiments, lighting system 10 may be disposed in/incorporated by a passenger service unit (PSU) for a row of seats. As such, a lighting system 10 could be provided for each row of an aircraft, as well as for each section of different sections of a given row of an aircraft.

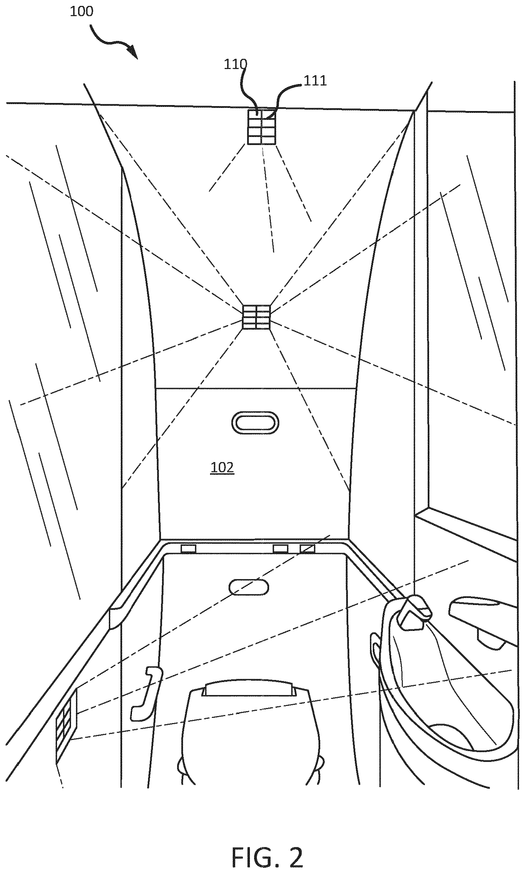

Referring now to FIG. 2, a perspective view of a sanitization system 100 is illustrated, in accordance with various embodiments. In various embodiments, the sanitization system 100 is disposed in a lavatory 102 of an aircraft cabin (e.g., aircraft cabin 51 from FIG. 1). The sanitization system 100 may comprise a light source 110 configured to emit a UV-C light. In this regard, the light source 110 may comprise an excimer lamp, a light emitting diode (LED), or the like configured to emit UV-C light. In various embodiments, the UV-C light may be directional light (i.e., oriented and directed in a specific direction) or it may be non-directional light. The present disclosure is not limited in this regard. The lavatory 102 may be configured to be sanitized during in-flight cycle, post-flight cycle, or the like. In various embodiments, the lavatory 102 may be configured to be sanitized after use (e.g., in response to detecting a user entering and detecting a user leaving). As described further herein, it may be beneficial to provide an indicator to a respective passenger that the lavatory 102 is being sanitized, in accordance with various embodiments.

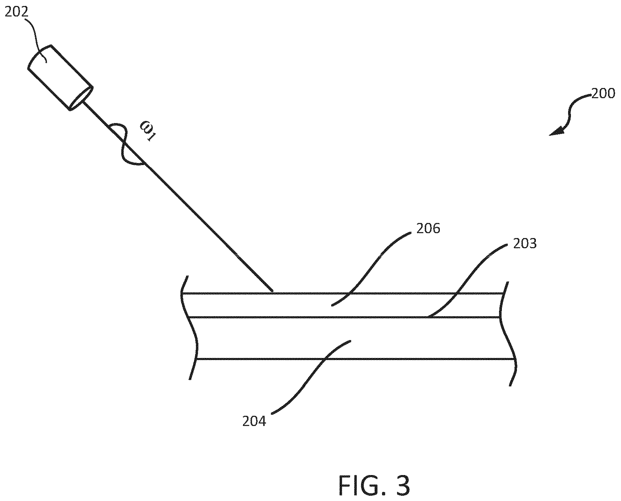

Referring now to FIG. 3, a schematic view of a sanitization indication system 200 is illustrated in accordance with various embodiments. The sanitization indication system 200 is configured to be used in a sanitization system 100 from FIG. 2, in conjunction with a sanitization lighting system 10 from FIG. 1, or the like. However, the sanitization indication system 200 is not limited in this regard. The sanitization indication system 200 may be utilized in any application where UV-C light is being utilized to sanitize a surface. For example, the sanitization indication system 200 may be utilized with a portable UV-C light, such as a sanitization wand, as described further herein, the sanitization indication system 200 may be utilized in any confined spaced configured for UV-C sanitization, etc.

In various embodiments, the sanitization indication system 200 comprises a light source 202. The light source 202 may comprise an excimer lamp, an LED, or the like. In various embodiments, the light source 202 is a component of a lighting system 10 from FIG. 1, a sanitization system 100 from FIG. 2 or the like. In various embodiments, the light source is disposed at a tip of a portable sanitization device.

In various embodiments, the sanitization indication system 200 comprises a surface 203 defined by a component 204. In various embodiments, the surface 203 may be a surface configured to be sanitized by a UV-C sanitization process described previously herein. In various embodiments, the surface 203 may comprise a surface that is not configured to be cleaned often (e.g., a wall). In this regard, the surface 203 may be strictly for indication purposes of a cleaning process being performed.

In various embodiments, the fluorescent brightener 206 may be configured to cover entirely the surface 203. Yet, the sanitization indication system 200 is not limited in this regard. For example, the fluorescent brightener 206 may be spaced apart on a surface to be sanitized, the fluorescent brightener 206 may depict an image indicating sanitization of a space is occurring, the fluorescent brightener 206 may spell out something on the surface 203 indicating a room or surface is being sanitized, or the like.

In various embodiments, the sanitization indication system 200 comprises a fluorescent brightener 206 disposed on the surface 203. The fluorescent brightener 206 may be configured to glow in response to reflecting UV-C light (i.e., light with a wavelength between 200 nm and 280 nm), in accordance with various embodiments. "Glow" as described herein refers to giving out steady visible light. "Visible light" as described herein refers to light having a wavelength between 380 nm and 750 nm, in accordance with various embodiments. In various embodiments, invisible UV-C light may be illuminated to provide an indication to an individual that a room, or a surface is being sanitized. In various embodiments, the fluorescent brightener 206 is configured to be excited in response to reflecting UV-C light (i.e., the excitement of the fluorescent brightener 206 results in the "glow").

In various embodiments, the fluorescent brightener 206 comprises an optical brightener, an optical brightening agent, a fluorescent brightening agent, or a fluorescent whitening agent (FWA). In various embodiments, the fluorescent brightener comprises one of triazine-stilbene, coumarins, imidazolines, diazoles, triazoles, benzoxazoles, biphenyl stilbenes, or the like. In various embodiments, the fluorescent brightener 206 is configured to absorb light in the UV-C light wavelength (i.e., 200 - 280 nm, or approximately 222 nm), and re-emit light in a visible wavelength (e.g., 420 - 470 nm, or any other visible light). In various embodiments, the fluorescent brightener 206 comprises a coating. In this regard, the fluorescent brightener 206 may be applied to any surface (e.g., surface 203) as described further herein.

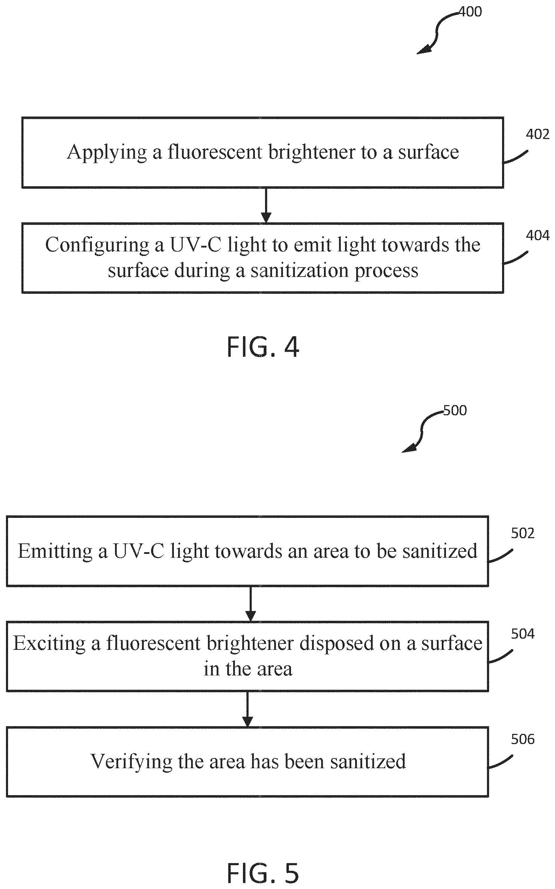

Referring now to FIG. 4, a process for using a sanitization indication system 200 is illustrated in accordance with various embodiments. The process 400 comprises applying a fluorescent brightener to a surface (step 402). The surface may be in accordance with surface 203 (i.e., a surface to be regularly sanitized or a surface to act as an indicator). In various embodiments, the fluorescent brightener may be applied as via coating, painting, dying, or the like.

In various embodiments, the process 400 further comprises configuring a UV-C light to emit light towards the surface during a sanitization process (step 404). In various embodiments, the UV-C light may be direction controlled to clean the surface from step 402. In this regard, the UV-C light may be sanitizing the surface and acting as an indicator the surface is being sanitized at the same time, in accordance with various embodiments. In various embodiments, the surface from step 402 may be strictly for indication purposes (e.g., on a wall across from a surface being sanitized). In various embodiments, the UV-C light may be directionless (e.g., an excimer lamp). In this regard, the surface from step 402 may also be used strictly for indication purposes and be within a range of the light source to be excited, in accordance with various embodiments.

Referring now to FIG. 5, a method of ensuring sanitization of an area is illustrated, in accordance with various embodiments. The method 500 comprises emitting a UV-C light towards an area to be sanitized (step 502) The area may include a surface having a fluorescent brightener disposed thereon. The fluorescent brightener may be disposed on the entire area. For example, a toilet seat may include a fluorescent brightener disposed on the area of the toilet seat, in accordance with various embodiments. In various embodiments, emitting the UV-C light may be performed via a portable UV-C sanitization system (e.g., a portable light source or the like). In this regard, sanitization may be performed by an individual via UV-C light being emitted from the portable UV-C sanitization system.

The method 500 further comprises, exciting the fluorescent brightener disposed on the surface in the area (step 504). The fluorescent brightener may be applied in accordance with process 400 from FIG. 4. In various embodiments, a user of the portable sanitization system may receive an indication from the fluorescent brightener that the surface is in fact being sanitized (i.e., that the user is sanitizing an intended target) in response to exciting the fluorescent brightener. For example, the fluorescent brightener absorbs invisible UV-C light, is excited in step 504, and re-emits visible light (e.g., blue light between 400 and 470 nm or the like), in accordance with various embodiments. Thus, the user may verify the area being sanitized throughout a sanitization process (step 506). In other words, the user may traverse the area to be sanitized and verify throughout in accordance with step 504 whether a respective surface is being sanitized.

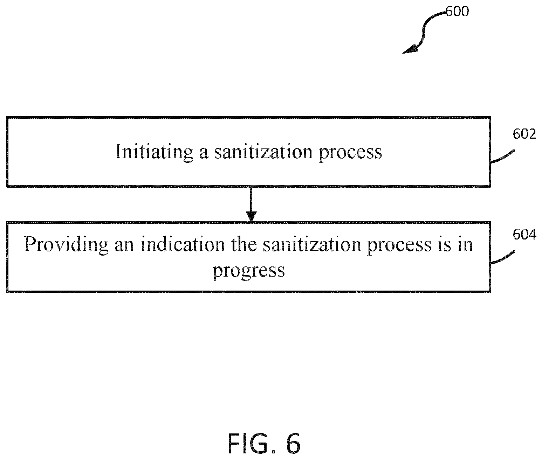

Referring now to FIG. 6, a method of providing an indication an area is being sanitized is illustrated in accordance with various embodiments. The method 600 comprises initiating a sanitization process (step 602). The sanitization process may include directing a beam of UV-C light towards surfaces to be sanitized, in accordance with various embodiments. In various embodiments, the sanitization process may include a directionless application of UV-C light (e.g., via an excimer lamp or the like).

The method 600 further comprising providing an indication the sanitization process is in progress (step 604). In various embodiments, the indication is as described previously herein. In other words, a fluorescent brightener may be disposed on a surface, absorb the UV-C light from step 602, and re-emit visible light. The indication may allow the public to realize, upon entering an area, or room, being sanitized that the sanitization process is in progress.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment.

Finally, it should be understood that any of the above described concepts can be used alone or in combination with any or all of the other above described concepts. Although various embodiments have been disclosed and described, one of ordinary skill in this art would recognize that certain modifications would come within the scope of this disclosure. Accordingly, the description is not intended to be exhaustive or to limit the principles described or illustrated herein to any precise form. Many modifications and variations are possible within the disclosure as recited in the appended claims.

## Claims

1. A method of sanitization indication, the method comprising:
initiating (602) a sanitization process for a lavatory of an aircraft including emitting a UV-C light from a light source in a sanitization system; and
providing (604) an indication, by an indicator including a wording indicating sanitization of the lavatory, that the sanitization process is in progress, the indication being provided by a fluorescent brightener of the indicator disposed on a surface (203).

2. The method of claim 1, wherein the fluorescent brightener is configured to:
absorb the UV-C light; and
re-emit a visible light to indicate an area is being sanitized.

3. The method of claim 1 or 2, wherein a sanitized area is a lavatory of an aircraft.

4. The method of claim 1 or 2 wherein the surface (203) is on a wall in an aircraft lavatory being sanitized.

5. The method of claim 1 or 2, wherein the surface (203) is a desired surface (203) to be sanitized in an aircraft lavatory.

6. The method of any of claims 1 to 5, wherein the UV-C light includes a first wavelength between 200 nm and 280 nm.

7. The method of claim 6, wherein the visible light includes a second wavelength between 380 nm and 750 nm.

8. The method of any of claims 1 to 7, further comprising directing the UV-C light towards the surface (203) throughout the sanitization process.

9. A sanitization indication system for a lavatory of an aircraft, comprising:
a surface (203) configured to be disposed on a wall in the lavatory of the aircraft;
an indicator including a wording indicating sanitization of the lavatory, the indicator including a fluorescent brightener disposed on the surface (203), the fluorescent brightener configured to receive a UV-C light from a UV-C light source during a sanitization process.

10. The sanitization indication system of claim 9, further comprising the UV-C light source.

11. The sanitization indication system of claim 9 or 10, wherein the surface (203) is disposed across from a second surface (203) to be sanitized, the surface (203) configured to act solely as an indication of the sanitization process.

12. The sanitization indication system of any of claims 9 to 11, wherein the UV-C light is configured to emit the UV-C light that includes a first wavelength between 200 nm and 280 nm.

13. The sanitization indication system of claim 12, wherein the fluorescent brightener is configured to absorb the UV-C light and re-emit a visible light having a second wavelength between 380 nm and 750 nm.

## Patentansprüche

1. Verfahren zur Angabe von Hygienemaßnahmen, wobei das Verfahren Folgendes umfasst:
Einleiten (602) eines Prozesses für Hygienemaßnahmen für einen Waschraum eines Luftfahrzeugs, einschließlich Emittieren von UV-C-Licht von einer Lichtquelle in einem System für Hygienemaßnahmen; und
Bereitstellen (604) einer Angabe durch einen Indikator, der einen Text beinhaltet, der die Hygienemaßnahmen des Waschraums angibt, dass der Prozess für Hygienemaßnahmen im Gange ist, wobei die Angabe durch einen fluoreszierenden Aufheller des Indikators bereitgestellt wird, der auf einer Fläche (203) angeordnet ist.

2. Verfahren nach Anspruch 1, wobei der fluoreszierende Aufheller zu Folgendem konfiguriert ist:
Absorbieren des UV-C-Lichts; und
erneutes Emittieren eines sichtbaren Lichts, um anzugeben, dass ein Bereich desinfiziert wird.

3. Verfahren nach Anspruch 1 oder 2, wobei ein desinfizierter Bereich ein Waschraum eines Luftfahrzeugs ist.

4. Verfahren nach Anspruch 1 oder 2, wobei sich die Fläche (203) an einer Wand in einem Luftfahrzeugwaschraum befindet, der desinfiziert wird.

5. Verfahren nach Anspruch 1 oder 2, wobei die Fläche (203) eine gewünschte zu desinfizierende Fläche (203) in einem Luftfahrzeugwaschraum ist.

6. Verfahren nach einem der Ansprüche 1 bis 5, wobei das UV-C-Licht eine erste Wellenlänge zwischen 200 nm und 280 nm beinhaltet.

7. Verfahren nach Anspruch 6, wobei das sichtbare Licht eine zweite Wellenlänge zwischen 380 nm und 750 nm beinhaltet.

8. Verfahren nach einem der Ansprüche 1 bis 7, ferner umfassend Richten des UV-C-Lichts in Richtung der Fläche (203) während des gesamten Prozesses für Hygienemaßnahmen.

9. System zur Angabe von Hygienemaßnahmen für einen Waschraum eines Luftfahrzeugs, umfassend:
eine Fläche (203), die dazu konfiguriert ist, an einer Wand in dem Waschraum des Luftfahrzeugs angeordnet zu sein;
einen Indikator, der einen Text beinhaltet, der Hygienemaßnahmen des Waschraums angibt, wobei der Indikator einen auf der Fläche (203) angeordneten fluoreszierenden Aufheller beinhaltet, wobei der fluoreszierende Aufheller dazu konfiguriert ist, während eines Prozesses für Hygienemaßnahmen ein UV-C-Licht von einer UV-C-Lichtquelle zu empfangen.

10. System zur Angabe von Hygienemaßnahmen nach Anspruch 9, ferner umfassend die UV-C-Lichtquelle.

11. System zur Angabe von Hygienemaßnahmen nach Anspruch 9 oder 10, wobei die Fläche (203) gegenüber einer zweiten zu desinfizierenden Fläche (203) angeordnet ist, wobei die Fläche (203) dazu konfiguriert ist, ausschließlich als eine Angabe des Prozesses für Hygienemaßnahmen zu dienen.

12. System zur Angabe von Hygienemaßnahmen nach einem der Ansprüche 9 bis 11, wobei das UV-C-Licht dazu konfiguriert ist, das UV-C-Licht zu emittieren, das eine erste Wellenlänge zwischen 200 nm und 280 nm beinhaltet.

13. System zur Angabe von Hygienemaßnahmen nach Anspruch 12, wobei der fluoreszierende Aufheller dazu konfiguriert ist, das UV-C-Licht zu absorbieren und ein sichtbares Licht erneut zu emittieren, das eine zweite Wellenlänge zwischen 380 nm und 750 nm aufweist.

## Revendications

1. Procédé d'indication de désinfection, le procédé comprenant :
le déclenchement (602) d'un processus de désinfection d'un cabinet de toilette d'aéronef, comprenant l'émission d'une lumière UV-C à partir d'une source lumineuse d'un système de désinfection ; et
la fourniture (604) d'une indication, par un indicateur comprenant un libellé indiquant la désinfection du cabinet de toilette, que le processus de désinfection est en cours, l'indication étant fournie par un azurant optique de l'indicateur disposé sur une surface (203).

2. Procédé selon la revendication 1, dans lequel l'azurant optique est configuré pour :
absorber la lumière UV-C ; et
réémettre une lumière visible afin d'indiquer qu'une zone est en cours de désinfection.

3. Procédé selon la revendication 1 ou 2, dans lequel la zone désinfectée est un cabinet de toilette d'aéronef.

4. Procédé selon la revendication 1 ou 2, dans lequel la surface (203) est disposée sur une paroi d'un cabinet de toilette d'aéronef en cours de désinfection.

5. Procédé selon la revendication 1 ou 2, dans lequel la surface (203) est une surface souhaitée (203) à désinfecter dans un cabinet de toilette d'aéronef.

6. Procédé selon l'une quelconque des revendications 1 à 5, dans lequel la lumière UV-C comprend une première longueur d'onde comprise entre 200 nm et 280 nm.

7. Procédé selon la revendication 6, dans lequel la lumière visible comprend une deuxième longueur d'onde comprise entre 380 nm et 750 nm.

8. Procédé selon l'une quelconque des revendications 1 à 7, comprenant en outre la direction de la lumière UV-C vers la surface (203) pendant toute la durée du processus de désinfection.

9. Système d'indication de désinfection pour un cabinet de toilette d'aéronef, comprenant :
une surface (203) configurée pour être disposée sur une paroi du cabinet de toilette de l'aéronef ;
un indicateur comprenant un libellé indiquant la désinfection du cabinet de toilette, l'indicateur comprenant un azurant optique disposé sur la surface (203), l'azurant optique étant configuré pour recevoir une lumière UV-C provenant d'une source de lumière UV-C pendant un processus de désinfection.

10. Système d'indication de désinfection selon la revendication 9, comprenant en outre la source de lumière UV-C.

11. Système d'indication de désinfection selon la revendication 9 ou 10, dans lequel la surface (203) est disposée en vis-à-vis d'une seconde surface (203) à désinfecter, la surface (203) étant configurée pour agir uniquement comme indication du processus de désinfection.

12. Système d'indication de désinfection selon l'une quelconque des revendications 9 à 11, dans lequel la lumière UV-C est configurée pour émettre une lumière UV-C comprenant une première longueur d'onde comprise entre 200 nm et 280 nm.

13. Système d'indication de désinfection selon la revendication 12, dans lequel l'azurant optique est configuré pour absorber la lumière UV-C et réémettre une lumière visible ayant une deuxième longueur d'onde comprise entre 380 nm et 750 nm.
